Europäisches Patentamt

European Patent Office

Office européen des brevets

(19)

(11) Veröffentlichungsnummer: **0 191 154**
A2

(12) ## EUROPÄISCHE PATENTANMELDUNG

(21) Anmeldenummer: 85114753.8

(22) Anmeldetag: 21.11.85

(51) Int. Cl.⁴: **A 61 M 25/00**

(30) Priorität: 12.02.85 DE 3504661

(43) Veröffentlichungstag der Anmeldung: 20.08.86
Patentblatt 86/34

(84) Benannte Vertragsstaaten: **AT BE CH DE FR GB IT LI LU NL SE**

(71) Anmelder: **CYTOMED Medizintechnik GmbH, Fabrikstrasse 20, D-8750 Aschaffenburg (DE)**

(72) Erfinder: **Gessler, Reiner, Glasbergstrasse 44, D-8752 Daxberg (DE)**

(74) Vertreter: **Patentanwälte Dr. Solf & Zapf, Asamstrasse 8, D-8000 München 90 (DE)**

(54) **Katheter für die Dauerapplikation von Pharmaka.**

(57) Die Erfindung betrifft einen Katheter für die Dauerapplikation von Pharmaka mit einem an der Katheterspitze angeordneten Einwegeventil, welches als Entenschnabelventil ausgebildet ist. Dasselbe weist vorzugsweise zur Befestigung am Katheterschlauch einen Stutzen auf, der auf oder in den Katheterschlauch geschoben wird und entweder aufgrund seiner Elastizität oder durch Verkleben oder Verschweißen haftet.

EP 0 191 154 A2

ł

## Katheter für die Dauerapplikation von Pharmaka

Die Erfindung betrifft einen Katheter für die Dauerapplikation von Pharmaka.

Ein solcher Katheter findet zum Beispiel in der modernen Krebs- bzw. Schmerztherapie Anwendung und gestattet die intermittierende Injektion bzw. Infusion von Medikamenten und Lösungen, die beispielsweise intraarteriell den befallenen Organen direkt zugeführt werden können. Ein solcher Katheter eignet sich dabei sowohl für stationären, als auch für ambulanten Einsatz.

0191154

Der Katheter, der in das ausgewählte Gefäß (beispielsweise Arterie) eingeführt wird, weist ein sogenanntes Einwegeventil auf, welches den Durchtritt der injizierten Medikamente bzw. Lösungen in die Gefäße zuläßt, andererseits jedoch verhindert, daß Flüssigkeiten aus den Gefäßen in den Katheter zurückfließen können.

Bei den bisher bekannten Ausführungsformen, bei denen das Ventil (falls vorhanden) hinter der Katheterspitze angeordnet ist (in diesem Fall ist die Katheterspitze verschmolzen), ist das verwendete Ventil ähnlich einem sogenannten Fahrradventil ausgebildet, d.h. der Katheter ist hinter der Spitze mit einer oder mehreren seitlichen Öffnungen versehen, die von einem dünnwandigen elastomeren Schlauchteil abgedeckt sind. Wird die Substanz in den Katheter injiziert, so bewirkt der dabei erzeugte Überdruck, daß sich die obengenannte Schlauchabdeckung von den seitlichen Öffnungen abhebt und somit den Durchtritt der Substanz zuläßt. Läßt der Druck nach, schließt das dünnwandige Schlauchteil aufgrund seiner Elastizität die hinter der Katheterspitze angeordneten seitlichen Öffnungen und verhindert so einen Rückstrom der Körperflüssigkeit in den Katheter.

Bei dieser bereits bekannten Ausführung erweist sich die Ausführung des Einwegeventiles hinter der Katheterspitze als problematisch.

Einerseits soll das auf den Katheter aufgeschobene, die seitlichen Öffnungen verschließende, elastische Schlauchteil möglichst fest auf dem Katheter sitzen, damit es sich nicht während der Injektion bzw. Infusion von Pharmaka löst, andererseits bedingt das jedoch einen relativ festen Verschluß der an dem Katheter angebrachten seitlichen
Öffnungen, so daß ein relativ hoher Innendruck im Katheter erforderlich ist, bevor die seitlichen Öffnungen freigegeben werden, damit das injizierte bzw. infundierte Medium austreten und in das jeweilige Gefäß gelangen kann.

Weitere Risiken und Probleme bei den herkömmlichen obengenannten Kathetern bzw. bei solchen, bei denen ein Ventil gänzlich fehlt und die für den oben beschriebenen
Zweck verwendet werden, in Bezug auf unvollkommen angerundete Spitzen, ungleichmäßige Verrundung bzw. Verschmelzung der Katheterspitzen und nicht gratfrei ausgestanzte, seitliche Öffnungen, die u.a. zu Irritationen
bzw. Perforationen der Gefäßwände führen können, sind
durch einschlägige Fachliteratur bereits bekannt.

Diese Nachteile bei den bekannten Ausführungsformen zu
beseitigen und einen Katheter zu schaffen, der schon bei
geringem Überdruck einen Austritt der Medikamente und
Lösungen ermöglicht, den Katheter sicher in Bezug auf
rückströmende Körperflüssigkeiten verschließt und z.B.
Irritationen der Gefäßwände vermindert, ist Aufgabe der
vorliegenden Erfindung.

Gelöst wird diese Aufgabe erfindungsgemäß dadurch, daß
bei einem Katheter der eingangs definierten Art das Einwegeventil ein sogenanntes Entenschnabelventil ist, welches direkt an der Katheterspitze angeordnet ist.

Ein solches Entenschnabelventil benötigt nur einen relativ geringen Überdruck, um die im Katheter enthaltenen
Pharmaka austreten zu lassen. Andererseits verschließt
es die Katheteröffnung sofort, sobald der Druck im Inneren des Katheters geringer wird als der Druck der jeweiligen Körperflüssigkeit, so daß keine Gefahr besteht, daß Körperflüssigkeiten in den Katheter zurückströmen.

Es hat sich zumindest bei der Krebs- bzw. Tumortherapie als sehr zweckmäßig erwiesen, wenn das Entenschnabelventil mit einem das Katheterschlauchende umgreifenden Stutzen auf das Schlauchende aufgeschoben ist.

Der Stutzen, dessen Rand zweckmäßig abgerundet ist, dient dabei gleichzeitig zur Befestigung der Katheterspitze im entsprechenden Gefäß, welches zum befallenen Organ führt. Dasselbe wird geöffnet, die Katheterspitze eingesetzt und dann mit einer sogenannten Tabakbeutelnaht hinter dem Rand des Stutzens verschlossen, so daß die Katheterspitze fest im Gefäß verankert ist.

Gemäß einer anderen vorteilhaften Ausführungsform ist der das Schlauchende umgreifende Stutzen mit einem vorzugsweise abgerundeten Kragen versehen, der ebenfalls zur sicheren Befestigung in dem Gefäß beiträgt.

Gemäß einer anderen Ausführungsform der vorliegenden Erfindung ist das Entenschnabelventil mit einem in das Katheterschlauchende einsetzbaren Stutzen versehen, der in besonders vorteilhafter Weise mit einer als Anschlag für den Schlauch wirkenden, vorzugsweise abgerundeten Schulter versehen ist.

Der Vorteil gegenüber den herkömmlichen Kathetern der eingangs beschriebenen Art, die z.B. mit Wulst-, Kugel- bzw. Fahrradventilen ausgeführt sind, besteht in der Form, daß hier eine Möglichkeit geschaffen wird, den Katheter mit Entenschnabelventil (Ventil trägt nicht auf dem Katheter

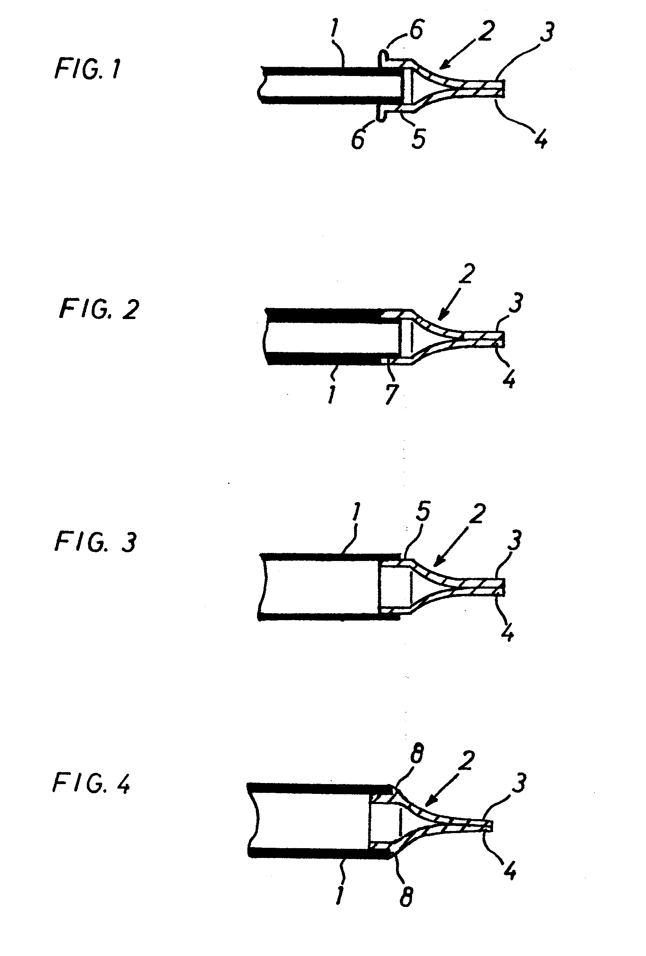

auf, wie in Fig. 2 - 4 dargestellt) auch in kleineren Gefäßen (z.B. Arterien) zu placieren, ohne daß die Gefahr
eines Verschlusses der Gefäße besteht, was unter Umständen
bei den zur Zeit auf dem Markt befindlichen Kathetern,
die mit Ventilen der oben aufgeführten Art ausgestattet
sind, der Fall sein kann.

Ein weiterer Vorteil des direkt an der Katheterspitze angebrachten Entenschnabelventils ist, daß bedingt durch das
hochflexible Ventilmaterial (vorzugsweise aus Silikonkautschuk oder dergleichen) die Möglichkeit der Gefäßperforation (Hämatombildung) und Irritationen der Gefäßwände
gegenüber herkömmlichen Kathetern dieser Art (wie bereits
beschrieben) praktisch ausgeschlossen werden.

Die Befestigung des Stutzens im oder auf dem Schlauch kann
einmal durch entsprechende Passung erfolgen dergestalt,
daß der in gewissem Maße elastische Schlauch auf den geringfügig dickeren Stutzen aufgeschoben wird und durch
seine Elastizität fest haftet oder aber in den Stutzen
eingeschoben wird, der einen etwas geringeren Durchmesser
als den Schlauchaußendurchmesser aufweist. Auch in diesem
Falle haftet der Schlauch aufgrund seiner Elastizität.

Die Haftung kann indessen gemäß einer vorteilhaften Ausführungsform der Erfindung weiterhin dadurch verbessert
werden, daß das Ventil durch Verkleben mit dem Schlauchende verbunden ist .

Gemäß einer anderen vorteilhaften Ausführungsform der vorliegenden Erfindung ist das Ventil mit seinem Stutzen
durch Hochfrequenzverschweißung mit dem Schlauchende verbunden.

Letzteres ist insofern besonders vorteilhaft, weil keine
besonderen Klebmaterialien erforderlich sind, die selbstverständlich ebenfalls blut- und gewebekompatibel sein
müssen.

Anhand der in den anliegenden Zeichnungen dargestellten
Ausführungsbeispiele wird nachfolgend die Erfindung im
einzelnen näher erläutert.

In den Zeichnungen zeigt:

Figur 1    das Katheterschlauchende mit dem daran befestigten Entenschnabelventil mit umlaufendem Kragen
           im Schnitt;

Figur 2    eine andere Art der Befestigung des Entenschnabelventils am Katheterschlauchende im Schnitt;

Figur 3    ein mit seinem Stutzen in das Katheterschlauchende eingesetztes Entenschnabelventil im Schnitt;

Figur 4    ein mit umlaufender Schulter versehenes Entenschnabelventil im Schnitt.

Das erfindungsgemäß am Katheterschlauchende 1 befestigte
Entenschnabelventil 2 ist ein an sich bekannter, aus
weich-elastischem Material, vorzugsweise Weichgummi, bestehender Ventilkörper, bei dem die beiden Schnabelflächen 3, 4 aufgrund der Formgebung aufeinander liegen und
das Ventil von außen her abdichten. Ein geringer Überdruck
im Katheterschlauch 1 bewirkt indessen, daß die beiden
aufeinander liegenden Schnabelflächen 3, 4 voneinander
abheben und den Durchtritt für das im Katheterschlauch 1
befindliche Medium freigeben. Läßt der Überdruck im Schlauch nach, so kehren die beiden Schnabelflächen 3, 4 aufgrund ihrer Elastizität wieder in ihre ursprüngliche aufeinander liegende Lage zurück und dichten das Schlauchende auch gegen einen größeren Überdruck von außen ab.

Bei der in Figur 1 dargestellten Ausführungsform ist das aus weichelastischem Material bestehende Entenschnabelventil 2 auf den Katheterschlauch 1 aufgeschoben, wobei sein Außendurchmesser etwas größer ist als der Innendurchmesser des Ventils, so daß letzteres aufgrund seiner Elastizität auf dem Schlauchende haftet.

Der Stutzen 5 des Ventils 2 ist dabei mit einem umlaufenden Kragen 6 versehen, der abgerundet sein kann und einmal zur besserer Haftung des Ventils auf dem Schlauchende beiträgt, andererseits aber auch zur Befestigung der so ausgebildeten Katheterspitze im Gefäß (z.B. Arterie, Vene) dient. Das Ventil wird mit dem Kragen 6 in das Gefäß eingeführt und z.B. mit einer sogenannten Tabakbeutelnaht hinter dem Kragen 6 wieder verschlossen.

Die Abrundung des Kragens 6 vermeidet dabei eine Reizung oder Beschädigung des Gefäßes. Zusätzlich zur Elastizität des Ventils 2 kann dasselbe zur besseren Haftung auf dem Schlauchende 1 verklebt oder verschweißt sein.

Bei der in Figur 2 gezeigten Ausführungsform ist das Katheterschlauchende 1 mit einer umlaufenden Abstufung 7 versehen, auf die das Entenschnabelventil 2 mit seinem Stutzen aufgeschoben wird und dort aufgrund seiner Elastizität oder durch Verkleben oder Verschweißen haftet.

Bei der Ausführungsform nach Figur 3 ist das Entenschnabelventil mit einem Stutzen 5 versehen, auf den das Katheterschlauchende 1 aufgeschoben wird. Bei einer solchen Ausführungsform ist der Stutzen 5 zweckmäßig auf seiner Innenseite mit einer Versteifung versehen, so daß die Elastizität des Schlauches 1 zur Befestigung ausgenutzt werden kann. Darüber hinaus kann die Befestigung auch durch Kleben oder Verschweißen erfolgen.

Bei der Ausführungsform nach Figur 4 ist am Stutzen 5 des Entenschnabelventils 2 eine umlaufende Schulter 8 vorgesehen, die dem Schlauchende 1 als Anschlag dient und so für eine glatte Oberfläche der Katheterspitze sorgt.

Patentansprüche

1.  Katheter für die Dauerapplikation von Pharmaka mit einem an der Katheterspitze angeordne-
    tem Einwegeventil , d a d u r c h   g e -
    k e n n z e i c h n e t ,  daß das Einwege-
    ventil als Entenschnabelventil (2) ausgebildet
    ist.

2.  Katheter nach Anspruch 1 ,  d a d u r c h
    g e k e n n z e i c h n e t ,  daß das Enten-
    schnabelventil (2) mit einem das Katheterschlauchende (1) umgreifenden Stutzen (5) auf
    das Schlauchende aufgeschoben ist.

3. Katheter nach Anspruch 2 , d a d u r c h  g e k e n n z e i c h n e t , daß der das Schlauchende (1) umgreifende Stutzen (5) mit einem Rand oder Kragen (6) versehen ist.

4. Katheter nach Anspruch 2 , d a d u r c h  g e k e n n z e i c h n e t , daß der das Schlauchende (1) umgreifende Stutzen (5) mit einem vorzugsweise abgerundeten Kragen (6) versehen ist.

5. Katheter nach Anspruch 1 , d a d u r c h  g e k e n n z e i c h n e t , daß das Enten-schnabelventil (2) mit einem in das Katheterschlauchende (1) einsetzbaren Stutzen (5) versehen ist.

6. Katheter nach Anspruch 5 , d a d u r c h  g e k e n n z e i c h n e t , daß der in das Katheterschlauchende (1) einsetzbare Stutzen (5) mit einer als Anschlag für den Schlauch wirkenden, vorzugsweise abgerundeten Schulter (8) versehen ist.

7. Katheter nach einem der Ansprüche 1 bis 6 , d a d u r c h  g e k e n n z e i c h n e t , daß das Entenschnabelventil (2) durch Verkleben mit dem Schlauchende (1) verbunden ist.

8. Katheter nach einem der Ansprüche 1 bis 6 , d a d u r c h  g e k e n n z e i c h n e t , daß das Entenschnabelventil (2) durch Verschweißung mit dem Schlauchende (1) verbunden ist.

0191154

FIG. 1

FIG. 2

FIG. 3

FIG. 4